# EUROPÄISCHE PATENTSCHRIFT

(11) **EP 0 914 138 B1**
(45) Veröffentlichungstag und Bekanntmachung des Hinweises auf die Patenterteilung: **12.03.2003**
(21) Anmeldenummer: 97927099.8
(22) Anmeldetag: 02.06.1997
(51) Int. Cl.: A61K 35/78, A61K 33/14, A61K 31/205, A61K 33/42

(54) **KOMPLEXE ZUBEREITUNGEN GEKENNZEICHNET DURCH EINEN GEHALT AN BETAIN**
COMPLEX PREPARATIONS CHARACTERISED BY A BETAIN CONTENT
PREPARATIONS COMPLEXES CARACTERISEES PAR UNE TENEUR EN BETAINE

(30) Priorität: 05.06.1996 DE 19622708; 21.11.1996 DE 19648232
(43) Veröffentlichungstag der Anmeldung: 12.05.1999
(73) Patentinhaber: Tomifarm S.r.l., 20122 Milano (IT)
(72) Erfinder: TOMIC, Dobrivoje, I-20122 Milano (IT)
(74) Vertreter: Bianchetti, Giuseppe, Prof.
(86) Internationale Anmeldenummer: EP9702849
(87) Internationale Veröffentlichungsnummer: WO97046246

(56) Entgegenhaltungen:
- DE-A- 3 816 442
- DATABASE WPI Section Ch, Week 8315, Derwent Publications Ltd., London, GB; Class D21, AN 83-35120K, XP002042723 & GB 2 106 386 A (BROOKE R) 13 April 1983

## Beschreibung

Die Erfindung betrifft komplexe Zubereitungen zur topischen, intrakorporalen oder oralen Behandlung, Beinflussung, Erhaltung und Verbesserung von Zellfunktion, zellularem Stoffwechsel, physiologischen Prozessen, Mikrozirkulation, Immunität, Homeostase und/oder Vorbeugung, Heilung, Pflege verschiedener gewebsschädigender Manifestationen, Gewebsveränderungen und -deformationen, hervorgerufen/entstanden durch exogene und/oder endogene Faktoren bei Mensch und Tier.

Es ist bekannt, dass es Mittel und Zubereitungen zur Behandlung und/oder Verbesserung der Zellfunktion, physiologischen Prozessen oder der Immunität gibt. Bekanntlich gibt es auch topische Mittel zur oberflächlichen Anregung der Durchblutung oder zur Heilung und Pflege des menschlichen und tierischen Körpers. Die Grundlage dieser Mittel, insbesondere bei den Dermatika, bilden in der Regel Antibiotika, Kortisone, Antimykotika und andere oft sehr bedenkliche Komponenten. Oft sind sie sogar wegen ihren Nebenwirkungen kontraindiziert, wie z. B. während der Schwangerschaft oder bei Säuglingen. Diese Mittel sind auch mit anderen Nachteilen verbunden, da sie, wenn überhaupt, nur bei sehr langer Anwendung und unterstützt durch andere Mittel, oder durch eine spezielle Diät, eine therapeutische Wirkung aufweisen. Keines dieser bekannten Mittel weist ionenhaltige Zubereitungen auf, welche unter hohem osmotischen Druck die enthaltenen physiologisch notwendigen Stoffe wie aminosäurehaltige Substanzen, Enzyme, Mineralstoffe, Vitamine u. a. durch die Einwirkung auf die Zellmembran ins Zellinnere und Blutplasma einschleusen können.

Aufgabe der Erfindung ist es, Zubereitungen mit natürlichen Stoffen auf phytobiologischer Basis, insbesondere für die topische Anwendung zur raschen wirksamen Behandlung, Beinflussung, Erhaltung der Zellfunktion, des zellularem Stoffwechsels, der physiologischen Prozesse, der Mikrozirkulation, Immunität, Abwehrkraft, Homeostase und/oder Vorbeugung, Heilung, Pflege verschiedener gewebsschädigender Manifestationen, Gewebsveränderungen und -deformationen, enstanden oder entstehend durch exogene und/oder endogene Einflüsse bei Mensch und Tier, bereitzustellen, welche die Ionen und Wirkstoffe unter hohem osmotischen Druck in die Gewebe bis in die Zellschicht bringen, die Zellmembran selektiv durchlässig machen und ins Zellinnere und Blutplasma gelangen und mit den notwendigen Substanzen, Mineralstoffen, Aminosäuren, Enzymen, Vitaminen, Proteinen, Sauerstoff und/oder anderen Komponenten versorgen und signifikant zur Unterstützung und Förderung physiologischer Vorgänge und zur Wiederherstellung des Gleichgewichtes im Organismus beitragen.

Diese Aufgabe wird durch die erfindungsgemässen Zubereitungen gelöst. Der Stand der Technik kennt ein topisches Mittel zur Behandlung mykotischer, mikrobischer Gewebsschäden (DE 3816442 C2).

Außerdem, UK Patentanmeldung n. 2 106 386 betrifft eine Zusammensetzung zur Kopfhaut- und Haarbehandlung, die eine Mischung von Metallen (insbesonders Eisen-, Magnesium-, Mangan-, Calcium-, Zinc- und Kupfersalzen), Phosphaten, Kohlenhydraten, Aminosäuren, Vitaminen und Ölen enthält.

Der Erfindung liegen spezielle Zusammensetzungen der Zubereitungen zugrunde. Die erfindungsgemässen Zubereitungen bestehen aus ionenhaltigen Verbindungen, Salzen, adstringierenden Komponenten, Amphotensiden, Feuchtigkeitsmitteln, Binde- und Haftmitteln, aetherischen Oelen, denen gegebenfalls ein oder mehrere zweckentsprechende Stoffe wie Enzyme, Vitämine, Proteine und Hilfskomponenten beigefügt werden können. Die erfindungsgemässen Zubereitungen sind gekennzeichnet nich nur durch die Anwesenheit von ionenhaltigen Verbindungen, aber besonders aurch die Anwesenheit von lipotropen Wirkstoffen und Betain. Als ionenhaltige Verbindungen und Salze werden die mit Na, Cl, K, Mg, Ca und Phosphat bevorzugt; als Adstringent vorzugsweise Tannin, Asparaginsäure, und/oder Sandelholzöl; als Feuchtigkeitsmittel Glyzerin und als aetherisches Oel bevorzugt Pfefferminzöl. Als Bindemittel eignet sich unter anderem vorzugsweise Pektin und/oder ein Hydrogel wie z. B. Agargel.

Die erfindungsgemassen Zubereitungen weisen einen vergleichsweise niedrigen bis physiologisch normalen pH-Wert und einen vergleichsweise hohen osmotischen Druck auf. Die erfindungsgemäss zusammengesetzten Komponenten weisen einen überraschend wirksamen synergistischen Effekt auf, wobei die Zellfunktion, der Stoffwechsel, die physiologischen Prozesse und die Mikrozirkulation bei gleichzeitigen Zufuhr und Versorgung aller Gewebe mit wichtigen Substanzen wie Mineralstoffen, Aminosäuren, Dextrose, Enzymen, Vitaminen, Proteinen, gefördert werden, was unter anderem besonders bei körperlichen Anstrengungen und gestörtem physiologischen Gleichgewicht sehr vorteilhaft ist.

In den erfindungsgemässen Zubereitungen können gegebenenfalls auch pflanzliche Extrakte, Emulgatoren, Aminosäuren, Lanolin, Glycin, Cholin, Eucerin, Farbstoffe, Wachse, Bakteriostatika, Oele, Starke, Zinn, Eisen, Fette, Gelatine, Konservierungsmittel, Vaseline, Kampfer, Harnstoff, Tenside, Paraffine, u. a. enthalten sein. Der Mengenanteil unter den Komponenten ist unterschiedlich und kann für eine Komponente 0,01 % und für eine andere 20 % und mehr betragen.

Die angegebenen Beispiele erläutern dies näher.

Die erfindungsgemässen Zubereitungen können in ihrer Konsistenz und Darreichungsform eine infundibile und/oder topische Lösung, Salbe, Creme, Gel, Lotion, Puder, Spray, Schaum, Emulsion, Tabletten, Kapseln, textile Wundauflagen, Verbandsmaterial oder teils/gänzlich vermischt mit anderen Mitteln und/oder Stoffen sein. Bei den erfindungsgemässen Zubereitungen können die angegebenen Anteile quantitativ nach oben oder unten schwanken und/oder es können Komponenten ausgelassen und/oder ausgetauscht werden.

Die erfindungsgemässen Zubereitungen unterscheiden sich in ihrer Zusammensetzung und dem daraus resultierenden Wirkungsprinzip und -effekt, Wirkungsablauf, Anwendungsmöglichkeiten, Verträglichkeit und Wirtschaftlichkeit grundlegend von allen vergleichbaren bekannten Mitteln, insbesondere von solchen,die für die topische Anwendung zur Beeinflussung, Förderung, Erhaltung, und/oder Wiederherstellung der Zellfunktion, der physiologischen Prozesse, der Mikrozirkulation, Immunität im menschlichen und tierischen Gewebe, besorgen.

Als lipotroper Wirkstoff ist Betain als Bestandteil zur Herstellung von schonenden, oberflächenaktiven, keimhemmenden Körperreinigungsmittel bekannt.

Es ist aber als höchst überraschend anzusehen, dass Betain als lipotrope, aminosäurehaltige Substanz in den erfindungsgemässen ionenhaltigen Zubereitungen mittels der Ionen und unter hohem osmotischen Druck die tiefe Zellenschicht erreicht, einen unerwartet hohen Zellen - und Nahrstoffaustausch, eine verbesserte Mikrozirkulation und umfangreiche physiologische Vorgange bewirkt. Es ist als überraschend anzusehen, dass das Betain nur in den angegebenen erfindungsgemässen Zubereitungen in die Gewebstiefe dringt und einen wirkunsvollen Einfluss auf die zellulären und physiologischen Vorgänge aufweist.

Es ist als überraschend anzusehen, dass man mittels der erfindungsgemässen ionenhaltigen Zubereitungen mit hohem osmotischen Druck wirkungsvolle Substanzen wie Betain, aber auch Enzyme, Vitamine, und andere lebenswichtigen Stoffe, und Elemente, in topischer Form rasch und gezielt verabreichen kann.

Die klinischen Ergebnisse bestätigen diese überraschenden Wirkungen und zeigen eine signifikant verbesserte Mikrozirkulation und verbesserte, gesteigerte physiologische Prozesse auf, die sich besonders an Haut - und Muskelgewebe, Sehnen, Gelenken, bei Heilungsprozessen, manifestieren. Diese überraschenden Ergebnisse können auch neue Wege und Vorteile im immunologischen, hämatologischen und metabolischen Bereich öffnen.

Die überraschende Wirkung der erfindungsgemässen betainhaltigen Zubereitung wurde durch folgende klinischen Tests bestätigt:

Fünfzehn Probandinnen im Alter von 20 bis 58 Jahren wurden in drei Gruppen (A, B, und C) zu je fünf Personen aufgeteilt. Alle Probandinnen wiesen an den Oberschenkeln das charakteristische Erscheinungsbild der Cellulitis im zweiten und dritten Stadium auf: Kneiftest positiv, matratzenformige Wölbungen im Stehen (zweites Stadium) oder sowohl im Stehen als auch im Liegen (drittes Stadium) mit blossem Auge sichtbar, Hauttonus schlaff. Diese Erscheinungen sind Folge einer mangelnden, gestörten Zellfunktion und Mikrozirkulation, die zu einer Wasser- und Fettansammlung im Gewebe führt. Die Probandinnen wurden dazu aufgefordert, während der Probezeit ihre Lebensgewohnheiten nicht zu ändern und keine Diäten oder Gymnastik/sportliche Aktivitäten zu beginnen.
**Gruppe A** hat auf die betröffen Hautflächen (rechter Oberschenkel) 14 Tage lang einmal täglich die erfindungsgemässe ionen-betainhaltige Zubereitung in einer dünnen Schicht aufgetragen und bis zur vollständigen Aufnahme der Haut einmassiert. Der Oberschenkelumfang wurde am ersten und letzten Behandlungstag an einer markierten Stelle gemessen. Schon nach vier Tagen wurde eine sichtbare Straffung und
Glättung der Haut festgestellt. Nach 8 Behandlungstagen war eine deutlich sichtbare Verringerung der Ausstülpungen an der Hautoberfläche zu bemerken. Nach 14 Tagen war die behandelte Hautfläche glatt, geschmeidig, elastisch und straff. Bei allen fünf Probandinnen wurde eine Abnahme des Oberschenkelumfangs von bis zu 3 cm gemessen.
**Gruppe B** hat die gleiche Behandlung mit einer ionenhaltigen Zubereitung wie unter A), jedoch ohne Beigabe von Betain durchgeführt. Nach ca. zehn Tagen wurde bei vier der fünf Probandinnen eine leichte Straffung und Glattung der Haut festgestellt. Nach der 14tägigen Behandlung zeigte sich jedoch keine weitere Veränderung oder Besserung des Erscheinungsbild der Cellulitis.
**Gruppe C** hat die gleiche Behandlung mit einem betainhaltigen Körperreinigungsmittel durchgefuhrt. Nach 14 Tagen wurde bei keiner der Probandinnen eine Besserung oder eine positive Veränderung festzustellen, weder was die Hautbeschaffenheit noch den Umfang des Oberschenkels betrifft.

| **Beispiel 1** | | |
|---|---|---|
| Komponente | Gehalt in gr. | ungefährer Gehalt in % |
| Betain | 1,0 | 0,1 |
| Hamamelis | 1,0 | 0,1 |
| Glyzerin | 20,0 | 2,0 |
| NaCl | 10,0 | 1,0 |
| MgCl | 0,8 | 0,08 |
| KCl | 0,8 | 0,08 |
| Na₄HPO₄·12H₂O | 6,0 | 0,6 |
| Agar | 2,0 | 0,2 |
| Tannin | 10,2 | 1,0 |
| Pfefferminzöl | 0,5 | 0,05 |
| Calendula | 1,0 | 0,1 |
| H2O | ad | 100,0 |

| **Beispiel 2** | | |
|---|---|---|
| Komponente | Gehalt in gr. | ungefährer Gehalt in % |
| Betain | 3,0 | 0,3 |
| Lanolin | 50 | 5,5 |
| Eucerin | 50 | 5,5 |
| Mandelöl | 100 | 10,0 |
| Paraffin | 100 | 10,0 |
| NaCl | 10,2 | 1,0 |
| MgCl | 0,8 | 0,08 |
| KCl | 0,8 | 0,08 |
| Tannin | 10,0 | 1,0 |
| Glyzerin | 20,0 | 2,0 |
| Vit. A + D | 0,13 | 0,01 |
| H₂O | ad | 100 |

| **Beispiel 3** | | |
|---|---|---|
| Komponente | Gehalt in gr. | ungefährer Gehalt in % |
| Betain | 10,0 | 1,0 |
| Harnstoff | 3,0 | 0,3 |
| Ca₃(PO₄)₂ | 80,0 | 8,0 |
| Glyzerin | 80,0 | 8,0 |
| Na₂HPO₄·12H₂O | 7,0 | 0,7 |
| NaCl | 6,5 | 0,6 |
| MgCl | 0,2 | 0,02 |
| KCl | 0,2 | 0,02 |
| Tannin | 12,0 | 1,2 |
| MgO₂ | 2,5 | 0,25 |
| Pektin | 80,0 | 8,0 |
| Pfefferminzöl | 1,0 | 0,1 |
| Benzalkoniumchlorid | 0,002 | 0,0002 |
| H2O | ad | 100,0 |

| **Beispiel 4** | | |
|---|---|---|
| Komponente | Gehalt in gr. | ungefährer Gehalt in % |
| Betain | 20,0 | 2,0 |
| Calendula | 0,5 | 0,05 |
| Hamamelis | 0,5 | 0,05 |
| Pektin | 30,0 | 3,0 |
| Tannin | 10,0 | 1,0 |
| Glyzerin | 20,0 | 2,0 |
| Na₂HPO₄·12H₂O | 6,0 | 0,6 |
| MgCl | 1,0 | 0,1 |
| KCl | 1,0 | 0,1 |
| NaCl | 10,0 | 1,0 |
| Pfefferminzöl | 0,5 | 0,05 |
| H₂O | ad | 100 |

| **Beispiel 5** | | |
|---|---|---|
| Komponente | Gehalt in gr. | ungefährer Gehalt in % |
| Betain | 20,0 | 2,0 |
| Avena sativa | 1,0 | 0,7 |
| Echinacea angustifolia | 1,0 | 0,1 |
| Urtica dioica | 1,0 | 0,1 |
| Pektin | 1,5 | 0,15 |
| NaCl | 0,3 | 0,03 |
| KCl | 0,3 | 0,03 |
| MgCl | 0,3 | 0,03 |
| Pfefferminzöl | 2,0 | 0,2 |
| CaHPO₄.2H₂O | 0,3 | 0,03 |
| Tannin | 0,2 | 0,02 |
| FeSO₄ | 0,02 | 0,002 |
| H₂O | ad | 100 |

| **Beispiel 6** | | |
|---|---|---|
| Komponente | Gehalt in gr. | ungefährer Gehalt in % |
| Betain | 60,0 | 6,0 |
| Pektin | 110,0 | 11,0 |
| Tannin | 15,0 | 1,5 |
| CaH(PO₄)₂ | 100,0 | 10,0 |
| Glyzerin | 115,0 | 11,5 |
| NaCl | 14,0 | 1,4 |
| MgCl | 1,0 | 0,1 |
| KCl | 1,0 | 0,1 |
| Na₂HPO₄·12H₂O | 12,0 | 1,2 |
| MgO₂ | 2,0 | 0,2 |
| Pfefferminzöl | 1,0 | 0,1 |
| Benzalkoniumchlorid | 0,002 | 0,0002 |
| H₂O | ad | 100,0 |

## Patentansprüche

1. Komplexe Zubereitungen mit folgenden Komponenten:
a) einer oder mehreren ionenhaltigen Verbindungen und lipotropen Substanzen
b) einem adstringierenden Mittel, einem Binde- und Haftmittel, einem Feuchtigkeitsmittel und einem oder mehreren aetherischen Oele
c) gegebenenfalls Aminosäuren, Proteinen, antimykotischen, entzündungshemmenden, pflanzlichen und sonstigen zweckentsprechenden Komponenten, **gekennzeichnet durch** einen Gehalt an Betain.

2. Zubereitungen nach Anspruch 1, **dadurch gekennzeichnet, dass** die Komponente a) Na-, K-, Cl-, Mg-, Ca und Phosphatverbindungen oder mindestens eine dieser Verbindungen enthält.

3. Zubereitungen nach Anspruch 1 und 2, **dadurch gekennzeichnet, dass** die Komponente b) als Adstringent Tannin oder eine sauere Verbindung, als Binde- und Haftmittel Pektin oder ein Gel, als aetherisches Oel Pfefferminzöl, oder mindestens eine dieser Komponenten enthält.

4. Zubereitungen nach Anspruch 1, 2 und 3, **dadurch gekennzeichnet, dass** die Komponente c) gegebenenfalls aminosäurehaltige Substanzen, säurehaltige Substanzen, lipotrope Substanzen, Bakteriostatika, Lanolin, Eucerin, pflanzliche Extrakte, Wachse, Farbstoffe, Oele, Starke, Zinn, Eisen, Sulphat-Verbindungen, Gelatine, Fette, Harnstoff, Tenside, Paraffine, Vaseline, Sandelholzoel, Kampfer, oder Konservierungsstoffe enthält.

5. Zubereitungen nach Anspruch 1 und 2, **dadurch gekennzeichnet, dass** sie mindestens 1,0 - 10 % Betain; 0,6 - 2,0 % NaCl; 0,02 - 0,5 % KCl; 0,02 - 0,5 % MgCl; 0,2 0,5 % MgO; 0,5 - 2,0 % Tannin; 2,0 - 20 % Glyzerin; 3,0 - 20 % Pektin; 0,6 - 2,0 % Na₂ HPO₄.12 H₂O; 3,0 - 20 % Ca₃ (PO4)₂; 0,1 - 0,6 % Pfefferminzöl enthalten.

6. Zubereitungen nach Anspruch 1 bis 5, **dadurch gekennzeichnet, dass** der pH-Wert 2,5 bis 7,8 beträgt und der osmotische Druck höher als 10 Atm ist.

7. Zubereitungen nach Anspruch 1-6, zur Behandlung, Beinflussung, Erhaltung und Verbesserung der Mikrozirkulation, Zellfunktion, physiologischer Prozesse, insbesondere an Haut- und Muskelgewebe, Sehnen, Gelenken, und zur Heilung, Pflege und Vorbeugung gewebsschädigender Vorgänge, Gewebs- und Körperdeformationen, Immunitätsstarkung und Zufuhr notwendiger Mineralstoffe, Vitamine, Enzyme und anderen Stoffen.

8. Zubereitungen nach Anspruch 1 bis 7, in Form einer Lösung, Paste, Creme, Emulsion, Gel, Salbe, Puder, Spray, Schaum, Gelatine, Tabletten, Kapseln, textilen Wundauflagen, Verbandsmaterial, auch teils oder gänzlich mit anderen Mitteln vermischt, zur topischen, intrakorporalen oder oralen Anwendung.

## Claims

1. Complex preparations containing the following components:
a) one or more ion-containing compounds and lipotropic agents
b) one astringent agent, one binding and adhesion agent, one hydrating agent and one or more etheric oils
c) optionally, aminoacids, proteins, antimicotic, anti-inflammatory, vegetable and other appropriate components,
**characterized in that** they contain betain.

2. Preparations according to Claim 1, **characterized in that** component a) contains Na-, K-, Cl-, Mg-, Ca- and phosphate-compounds, or at least one of such compounds.

3. Preparations according to Claims 1 and 2, **characterized in that** component b) contains tannin or an acid compound as astringent agent, pectin or a gel as binding and adhesion agent, peppermint oil as etheric oil, or at least one of such compounds.

4. Preparations according to Claims 1, 2 or 3, **characterized in that** component c) contains substances optionally containing aminoacids or acids, lipotropic agents, bacteriostatics, lanolin, eucerin, vegetable extracts, waxes, colouring agents, oils, starches, tin-, iron-, sulphate-compounds, gelatin, fats, urea, surfactants, paraffin, vaseline, sandalwood oil, camphor or preserving agents.

5. Preparations according to Claims 1 and 2, **characterized in that** they contain at least 1,0 - 10% betain, 0,6 - 2,0% NaCl; 0,02 - 0,5% KCl; 0,02 - 0,5% MgCl; 0,2 - 0,5% MgO; 0,5-2,0% tannin; 2,0 - 20% glicerin; 3,0 - 20% pectin; 0,6 - 2,0% Na₂ HPO₄.12 H₂O; 3,0 - 20% Ca₃ (PO4)₂; 0,1 - 0,6% peppermint oil.

6. Preparations according to claims 1-5, **characterized in that** the pH value is comprised between 2,5 - 7,8 and the osmotic pressure is higher than 10 Atm.

7. Preparations according to claims 1-6 for the treatment, control, maintenance and improvement of the microcirculation, cell functions, physiologic processes, with particular regard to skin and muscle tissues, tendons, joints, and for the healing, treatment and prevention of damaging processes in tissues, deformations of tissues and body, immunitarian reinforcement and supply of necessary minerals, vitamins, enzymes and other substances.

8. Preparations according to claims 1-7, in the form of solution, paste, cream, emulsion, gel, ointment, powder, spray, foam, gelatin, tablets, capsules, textile compresses, bandagings, also in combination, in whole or in part, with other agents, for topical, intracorporeal or oral use.

## Revendications

1. Préparations complexes ayant les suivants components:
a) un ou plusieurs composés ioniques et substances lipotropiques;
b) un agent astringent, un moyen liant et d'adhésion et un moyen humidifiant et un ou plusieurs huiles éthers;
c) éventuellement amino-acides, protéines, components antimycotiques, anti-inflammatoires, végétaux et d'autres components convenables
**caractérisées par** un contenu de bétaine.

2. Préparations selon la revendication 1, **caractérisées en ce que** le component a) contient des composés de Na, K, Cl, Mg, Ca et phosphate ou au moins un de ces composés.

3. Préparations selon les revendications 1 et 2, **caractérisées en ce que** le component b) contient tannin ou un composé acide comme astringent, pectine ou un gel comme moyen liant et d'adhesion, huile de menthe poivrée comme huile éther, ou au moins un de ces composés.

4. Préparations selon les revendications 1, 2 et 3, **caractérisées en ce que** le component c) contient des substances éventuellement contenant amino-acides, substances acides, substances lipotropiques, bactériostatiques, lanoline, eucerine, extraits végétaux, cires, colorants, huiles, amides, composés d'étain, fer, sulfate, gelées, lipides, urée, émulsifiants stabilisants, paraffines, vaseline, huile de bois de sandale ou substances de conservation.

5. Préparations selon les revendications 1 et 2, **caractérisées en ce qu'**elles contiennent au moins 1,0-10% de bétaine; 0,6-2,0% de NaCl; 0,02-0,5% de KCl; 0,02-0,5% de MgCl₂; 0,2-0,5% de MgO; 0,5-2,0% de tannin; 2,0-20% de glicerine; 3,0-20% de pectine; 0,6-2,0% de Na₂HPO₄.12H₂O; 3,0-20% de Ca₃(PO₄)₂; 0,1-0,6% de huile de menthe poivrée.

6. Préparations selon les revendications de 1 a 5, **caractérisées en ce que** le pH est de 2,5-7,8 et la pression osmotique est supérieure à 10 Atm.

7. Préparations selon les revendications de 1 a 6, pour traiter, influencer, maintenir et améliorer la micro-circulation, les fonctions cellulaires, les procédes physiologiques, en particulier sur le tissu epidermique et musculaire, tendons, articulations, et pour la thérapie, le soin et la prévention de procédes dommageables pour les tissus, de dèformation des tissus et du corps, pour renforcer l'immunité et apporter minéraux, vitamines, enzymes et d'autres substances nécessaires.

8. Préparations selon les revendications de 1 a 7, en forme de solution, pàte, crème, émulsion, gel, pomade, poudre, spray, écume, gelées, comprimés, cartouches, compresses de textiles, matériau de bandage, aussi bien partiellement que totalement melangés avec d'autres moyens, pour application topique, intracorporel ou oral.
